# EUROPEAN PATENT SPECIFICATION

(11) **EP 1 879 918 B1**
(45) Date of publication and mention of the grant of the patent: **08.05.2013**
(21) Application number: 06726519.9
(22) Date of filing: 28.03.2006
(51) Int. Cl.: C07K 14/705, G01N 33/569

(54) **METHOD OF PRODUCING A SET OF MHC MOLECULES**
VERFAHREN ZUR HERSTELLUNG EINES SATZES VON MHC-MOLEKÜLEN
METHODE D'OBTENTION D'UN ENSEMBLE DE MOLECULES CMH

(30) Priority: 01.04.2005 GB 0506706
(43) Date of publication of application: 23.01.2008
(73) Proprietor: ProImmune Limited, Oxford OX4 4GA (GB)
(72) Inventor: SCHWABE, Nikolai, Franz, Gregor, Oxford OX2 6BP (GB)
(74) Representative: Cremer & Cremer
(86) International application number: PCT/GB2006/001110
(87) International publication number: WO 2006/103406

(56) References cited:
- US-A- 5 763 585
- CAMERON T O ET AL: "Labeling antigen-specific CD4<+> T cells with class II MHC oligomers" JOURNAL OF IMMUNOLOGICAL METHODS, ELSEVIER, AMSTERDAM, NL, vol. 268, no. 1, 1 October 2002 (2002-10-01), pages 51-69, XP004379137 ISSN: 0022-1759
- GARBOCZI D N ET AL: "HLA-A2-peptide complexes: refolding and crystallization of molecules expressed in Escherichia coli and complexed with single antigenic peptides" PROCEEDINGS OF THE NATIONAL ACADEMY OF SCIENCES OF USA, NATIONAL ACADEMY OF SCIENCE, WASHINGTON, DC, US, vol. 89, no. 9, 15 April 1992 (1992-04-15), pages 3429-3433, XP002131059 ISSN: 0027-8424 cited in the application
- MALIK P ET AL: "Perfusion chromatography for very rapid purification of class I and II MHC proteins" JOURNAL OF IMMUNOLOGICAL METHODS, ELSEVIER SCIENCE PUBLISHERS B.V.,AMSTERDAM, NL, vol. 234, no. 1-2, February 2000 (2000-02), pages 83-88, XP004187659 ISSN: 0022-1759
- LATEK E.R., PETZOLD S.J., UNANUE E. R.: "Hindering auxiliary anchors are potent modulators of peptide binding and selectionby I-Ak class II molecules.", PROC. NATL. ACAD. SCI. USA, vol. 97, no. 21, 10 October 2000 (2000-10-10), pages 11460-11465,

## Description

The present invention relates to a method of producing a set of MHC molecules, and more precisely to a method of producing a set of MHC molecules which differ in their peptide bound in the binding groove. The method allows for parallel and expedite provision of differing MHC molecules at high yields for each molecule.

### Background of the Invention

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells. They consist of alpha and beta chain proteins, and a peptide bound in a groove formed by these chains when properly folded.

It has been established that isolated or recombinant forms of MHC-peptide molecules are useful for detecting, separating and manipulating T cells according to the specific peptide antigens these T cells recognize. It has also been understood that the interaction between MHC molecules and TCRs across cell surfaces is multimeric in nature and that the affinity of a single MHC molecule for a given TCR is generally quite low. As a consequence, there has been an effort to develop multimeric forms of isolated or recombinant MHC-peptide molecules that have an increased functional avidity in order to make such molecules more useful in the applications described above. Various methods are known from the art to purify protein complexes and especially MHC molecules.

European Patent Application EP 812 331 discloses a multimeric binding complex for labelling, detecting and separating mammalian T cells according to their antigen receptor specificity, the complex having the formula (α-β-P)ₙ, wherein (α-β-P) is an MHC peptide molecule, n is ≥ 2, α comprises an α chain of a MHC I or MHC II class molecule, β comprises a β chain of an MHC protein and P is a substantially homogeneous peptide antigen. The MHC peptide molecule is multimerised by biotinylating the C terminus of one of the α or β chain of the MHC molecule and coupling of MHC monomers to tetravalent streptavidin/avidin.

This method requires the site directed enzymatic biotinylation of the MHC molecules near one of its carboxyl termini. An enzyme recognition peptide sequence of around 14 amino acids is fused to the C-terminus of one MHC peptide chain proteins, which then allows for the complex to be biotinylated by using a biotinylating enzyme recognising this site. MHC alpha and beta chain proteins are refolded in the presence of peptide and then purified by column chromatography. Biotinylation is achieved by incubating purified MHC peptide complexes in a suitable buffer in the presence of the biotinylating enzyme BirA. However concentration steps before and after the chromatography steps are required for purification and changing the purified MHC molecules in and out of the buffer required for biotinylation. These steps usually lead to considerable losses in protein, and are hence typically carried out in a scale as large as possible.

Standard production of monomeric biotinylated MHC peptide complexes as described by the NIAID tetramer core facility as available on 31 March 2005 under the following URL: http:/lwww.yerkes.emory.edu/TETRAMER/pdf/Protocols.pdf involves concentration of typically 500ml of refold mixture on a stirred cell concentrator to approximately 7ml. This will be followed by buffer exchange into biotinylation buffer using a gravity driven desalting column. The MHC complexes are then biotinylated as described above. Purification is done thereafter first by size exclusion chromatography, which requires approximately 100 minutes run time. The recovered complexes are then concentrated in a centrifugal filtering device before loading onto an ion exchange chromatography column for final purification.

All prior art methods have in common that, if purification is disclosed at all, they rely on steps such as concentration and buffer exchange in their purification sequence. Such steps are associated with considerable protein losses. The fact that a variety of different concentration and chromatography steps have to be run in sequence makes the synthesis process for MHC monomers or oligomers time and labour intensive. This holds especially true for situations where a large number of differing MHC-peptide molecules is desired such as for T cell epitope characterization or validation applications.

It is the object of the invention to provide an improvement over the prior art by providing a fast and efficient method of producing a set of different MHC molecules, which method allows for high yields and lower losses in protein material.

### Summary of the Invention.

The above drawbacks and disadvantages of the prior art are overcome by the present invention, which in its first aspect relates to a method of producing a set of MHC molecules, which set includes a plurality of subsets of MHC molecules. The MHC molecules of each subset differ from MHC molecules of at least one, and preferably any other subset in at least one element selected from the group consisting of a peptide bound in the MHC binding groove, an MHC alpha chain protein and an MHC beta chain protein. Said method comprises the steps of:
a) providing the MHC molecules for each subset and loading said MHC molecules with said peptide by refolding MHC alpha and beta chain proteins of said MHC molecules in presence of said peptide, and
b) purifying substantially in parallel the subsets of MHC molecules as obtained in step a) by a chromatographic method using a stepped gradient for elution.

The above drawbacks and disadvantages of the prior art are overcome by the present invention, which in its second aspect relates to a method of producing a set of MHC molecules, which set includes a plurality of subsets of MHC molecules. The MHC molecules of each subset differ from MHC molecules of at least one, and preferably any other subset in at least one element selected from the group consisting of a peptide bound in the MHC binding groove, an MHC alpha chain protein and an MHC beta chain protein. Said method comprises the steps of:
a) providing the MHC molecules for each subset and loading said MHC molecules with said peptide by refolding in presence of said peptide or by peptide exchange, and
b) purifying substantially in parallel the subsets of MHC molecules as obtained in step a) by a chromatographic method using a stepped gradient for elution, wherein the set comprises at least four subsets of MHC molecules.

Preferably, the product of step a) is subjected to step b) without prior concentration.

### Detailed Description of the Invention

As described in the first and second aspects of the invention above, the drawbacks and disadvantages of the prior art are overcome by a method of producing a set of MHC molecules in purified form, each.

The methods of the invention - by avoiding concentration steps and by parallel processing of a plurality of subsets - allows for providing a set of MHC molecules at high yields and high diversity of the subsets. This makes available such sets of MHC molecules for screening applications at reasonable costs. In contrast the prior art processes essentially allow for producing one purified MHC molecule at a time. They are also carried out in rather large scale so that a sufficient amount of material can be recovered after all concentration, buffer exchange and purification losses. Both the requirement of large scale and high losses were up to now uneconomical for providing MHC molecules to be used in applications which may e.g. require evaluating 10 or more MHC molecules and possibly several 10s or even 100s of such molecules of e.g. differing specificity (as determined by differing peptides being bound in the MHC binding groove).

Usually when one studies many different proteins in an experimental setting and the study requires purification, such different protein species will have quite different physical properties and therefore have to be purified by different purification methods which may include a variety of chromatography methods. At least such proteins will be eluted under different conditions using the same purification method due to the difference in their physical properties.

The invention methods are based on the idea that various MHC molecules, though differing slightly e.g. in the peptide, are still usually very similar in their overall physical properties such as their isoelectric point, molecular weight, conformation and hydrophobicity. This allows their purification by applying essentially the same or very similar methods and conditions. This circumstance creates a unique opportunity for purifying MHC molecules of many different specificities of the individual subsets of MHC molecules substantially in parallel in a simplified but effective purification procedure.
Here, parallel processing reduces manufacturing and hence product costs.

In the context of this invention the term "substantially in parallel" means that the individual subsets are subjected to the same or similar process steps in close timely relationship, and preferably at substantially the same time or simultaneously. This does not require all of the subsets being processed at the same time, but is intended to include scenarios, wherein e.g. a first group of subsets is processed first and a second group is processed thereafter, and so on. Preferably, in parallel means a simultaneous processing of all subsets, even more preferably under similar or identical conditions. Parallel processing may e.g. be in the same device (such as a centrifuge or a vacuum manifold) or by using parallel-operated equipment.

Substantially parallel processing occurs essentially in step b), i.e. during purification. To meet this requirement at least one of the purification steps of loading, washing and elution is carried out in parallel. Typically at least elution/recovery is carried out in parallel. More preferably, at least washing and elution are parallel.

Providing the MHC molecules for each subset or loading of MHC molecules with peptide of step a) needs not be parallel for each subset, but can of course be in parallel, if technically feasible and/or desirable.

Since the MHC molecules will typically be provided in liquid solution in step a) the type of chromatography will usually be liquid chromatography.

The chromatographic method of step b) typically involves the steps of
(i) loading by contacting the surface of a chromatography adsorption matrix with a mixture of molecules in liquid solution, which solution includes a specific type of molecule to be purified under conditions so that at least the molecules to be purified will bind to the chromatography matrix,
(ii) optionally washing, if desired or necessary to achieve the intended purity, and
(iii) after such binding has occurred the step of elution by changing the composition of the solution in which the chromatograpy matrix is immersed over time until the molecules to be purified elute from the chromatography matrix.

According to the present invention elution is by using a stepped gradient. Using a stepped gradient for elution within the scope of the invention means changing the composition or property of the solution in which the chromatography matrix is immersed in one or more discrete steps as opposed to changing it continuously. For the purpose of distinguishing a continuous gradient from a stepped gradient relating to the invention, a continuous gradient may for example be created by analogue operation of a mixing valve or quasi-continuous change in mixing of two or more solvent components under digital computer control, such as in a computer operated liquid chromatography system. Hence, using a stepped gradient for elution will usually involve preparing separate aliquots of solutions with different properties (such as differing salt concentration) which may be applied in a step-wise sequence one after another until elution occurs. The number of steps in which this gradient is applied will depend on the chromatographic method chosen, tolerable losses and the desired purity. The stepped gradient will at least include one step and typically less than 50 steps, preferably less than 10 steps, more preferably less than 2 steps and most preferably only one step.

The MHC subsets differ from at least one, and preferably any other subset in at least one element selected from the group consisting of a peptide bound in the MHC binding groove, an MHC alpha chain protein and an MHC beta chain protein. The differing subsets taken together comprise the set to be provided by the present invention. Each subset may be processed in an individual batch. This limitation does not exclude the possibility of individual subsets being present in more than one batch, provided that the set includes at least two differing subsets.

Preferably the MHC molecules of the subsets differ with regard to their peptides and are loaded with a peptide specific for the respective subset in step a). Most preferably, said peptides are substantially homogenous in each subset. The set as produced may comprise at least 2, preferably at least 4 and more preferably at least 10 subsets of MHC molecules. It also preferably comprises up to 96, preferably up to 48 and more preferably up to 24 subsets of MHC molecules. The number of subsets may depend on the precise method of chromatography chosen and the device used for implementing the same.

Preferably, the chromatographic method is one using an adsorption matrix selected from the group consisting of resins, beads, and membranes (such as a porous membrane adsorption chromatography matrix). Column designs are preferred over batch designs (e.g. batch designs where the adsorption matrix is directly added to the receptacle containing the solution to be purified), due to ease of handling and availability of equipment. Various types of binding properties may be exploited such that the method may be chosen from the group consisting of affinity chromatography, ion exchange chromatography, iso-electric focussing chromatography, hydrophobic interaction chromatography, hydroxyl-apatite chromatography, and reverse phase chromatography; ion exchange chromatography being most preferred. In one preferred embodiment the adsorption matrix is a porous membrane adsorption matrix, such as described in WO0119483. More preferably such matrix is an ion exchange chromatography matrix.

The ion exchange chromatography method used is preferably anion exchange chromatography and most preferably uses a basic ion exchanger such as a resin comprising quaternary ammonium groups (DEAE). Commercial ion exchange resins suitable for use in the present invention are e.g. those sold under the trademarks Sepharose^{®}, MonoBeads^{®} from GE Healthcare (formerly Amersham Biosciences).

Preferably, the purification step b) comprises the steps of
b1) loading the product of steps a) on the adsorption matrix, e.g. the column,
b2) washing the loaded adsorption matrix (e.g. the column) at least once, and
b3) eluting the adsorption matrix (e.g. the column) by applying a stepped gradient,
b4) recovering the desired subset of MHC molecules in the eluate of step b3), and
b5) optionally repeating steps b1 to b4, individually or in combination, until the desired purity of the product is obtained.

Typically the methods of the invention will yield a purified set of MHC molecules, wherein impurities are removed from the MHC molecules in each subset during the wash step(s) before elution and/or by remaining bound to the matrix after the elution step(s). The nature of the impurities that typically remain on the column after the elution step(s) is usually larger protein aggregates. The methods of the invention also allow for efficient buffer exchange of the MHC molecules in each subset from the buffer solution that they are provided in in step a) to the buffer solution they are eluted in. At the same time the methods may serve as a simultaneous means of concentration of the MHC molecules in solution in each subset from the volume of solution that they are provided in in step a) to the volume of solution they are eluted in.

The stepped gradient is preferably selected from any suitable gradients depending on the chromatographic method chosen. It will typically be selected from the group consisting of a salt gradient, a pH gradient or a gradient employing a denaturing or chaotropic agent, and mixtures thereof. Most preferably the stepped gradient is a one-step gradient such as a one-step salt gradient.

The concentrations of the gradient are likewise chosen depending on the chromatographic method chosen and adsorption strength. For ion exchange chromatography preferably a salt gradient of at least 100 mM salt, preferably 200 to 500 mM salt is used. Most preferably the salt is NaCl.

The product of step a) is loaded on the adsorption matrix - in the following reference will be made to columns only, though it is to be understood that the same or comparable conditions will apply to other types of adsorption matrix as well - under any suitable conditions as to capacity, number of loading steps, buffer solution, flow rate, pH and temperature.

The column is then subjected to washing and elution. The solution used for at least one of the steps of purification selected from loading, washing and elution is any suitable solution such as an aqueous solution of pH above 7, preferably above 7.5. Identical solutions are typically chosen for the wash buffer and the loading buffer, but need not be. Elution occurs by using the stepped gradient as above. The entire sequence of purification steps in b) (b1 to b4) may be repeated individually (e.g. more than one washing step applied) or in combinations (sequence b1 to b4 is repeated), as desired.

Preferably, the conditions for carrying out at least one of the steps of purification in b) are substantially identical for at least two subsets and more preferably for all subsets.

In one embodiment the MHC molecules of each subset are provided and loaded with the desired peptide of interest in step a) by refolding MHC alpha and beta chain proteins of said MHC molecules in the presence such peptide as known in the art, e.g. by following a protocol described in Garboci et al., PNAS 89 (1992), 3429-3433 or the NIAID tetramer core facility protocol *(supra).* In both cases the MHC α and β chain proteins are expressed in a bacterial host and obtained from inclusion body material.

In an alternative embodiment the MHC molecules of each subset are provided in step a) by exchanging the peptide bound in said MHC molecules under suitable conditions to load the desired peptide as known in the art, such as disclosed in WO9310220 and T.O. Cameron et al., J. Immunol. Meth. 268 (2002), 51-69. In this case the MHC molecules for each subset are obtained by expression in a eukaryotic host and they may contain no peptide, peptide endogenous to the host cell or other irrelevant peptide before loading of the desired peptide of interest for each subset.

Hence depending on the embodiment chosen, providing the MHC molecules for each subset and loading with the said peptide of interest may take place simultaneously or in sequence.

According to the present invention the MHC molecules may be selected from MHC monomers and oligomers. The term oligomer is intended to include any molecule comprising more than one MHC monomer, each monomer consisting of alpha chain protein, beta chain protein and peptide bound in the binding groove. Examples of such oligomers are dimers to decamers, especially dimers, tetramers, pentamers and decamers. Oligomerization may occur as disclosed in the above references or in WO9310220, GB2392158 all of which are incorporated by reference.

In one embodiment the MHC molecules are MHC monomers, which have been biotinylated, preferably before subjecting them to step b). Preferably, the MHC monomers have been biotinylated on either their alpha or beta chain protein even before loading of the peptide either by refolding or peptide exchange in step a). Biotinylation of the MHC monomers can be achieved as known in the art, e.g. by attaching biotin to a specific attachment site which is the recognition site of a biotinylating enzyme. Reference is in this regard made e.g. to EP 812 331. In a preferred embodiment, biotinylation is carried out on the desired protein chain *in vivo* as a post translational modification during protein expression of such protein chain in the expressing host cells as described in WO9504069.

In case the desired MHC molecules in the set of the invention are MHC oligomers the method of the invention further includes the step of oligomerizing the MHC monomers to yield the desired oligomer. Such step of oligomerization may occur after purification. Alternatively it may occur before purification, either before step b) and after step a), or alternatively before or during step a).

The methods of the invention may further include a step of purifying the α and B chain proteins of the MHC molecules before providing the MHC molecules in step a). Purification of the α and β chain proteins as such or aligned to an MHC molecule may be carried out by any conventional method and preferably by ion exchange or affinity chromatography.

The driving force for at least one of the purification steps selected from loading, washing and elution is typically selected as appropriate for the method and device of chromatography chosen. It is preferably selected from gravitational forces, a centrifugal force applied by centrifuging the chromatography column or a pressure drop caused through applying a vacuum to one end of the column.

Preferably, the plurality of subsets is purified substantially simultaneously in the same centrifuge or on a vacuum manifold. Corresponding vacuum manifolds are known in the art and are commercially available e.g. from Qiagen under the trade name QIAvac 24 plus, which is a 24 place manifold. Alternatively, a commercially available centrifuge is used for spinning of appropriate columns.

Especially for processing of very small-scale samples it is preferred that the chromatography column is mounted directly on the receptacle receiving the eluate during the elution step b4). Corresponding spinning columns are commercially available e.g. from Vivascience under the trademark VivaPure^{®} such as the VivaPure^{®} Q Mini H column. The receptacle may also be a microcentrifuge tube suitable for holding between 0.5 to 2 ml volumes of solution such as those known in the art as Eppendorf^{®} tubes.

The methods of the invention are also characterised in that preferably no concentration steps are carried out before subjecting the product obtained from step a), i.e. the peptide loaded MHC molecule (monomer or oligomer) to the chromatography of the step b), and especially before loading the product of step a) onto the adsorption matrix. More preferably, none of a separate concentration or separate buffer exchange step is carried out between step a) and step b). In other words, the product of step a) is subjected to step b) essentially as is or after an oligomerization thereof.

The product obtained in step b) may further be subjected to one or more steps selected from a labelling reaction, lyophilization, immobilisation on a solid surface or incorporation into a lipid (bi)layer or quality control e.g. by SDS-PAGE, immune precipitation or other. Any of these steps may likewise be carried out in parallel for the subsets, but need not be so.

As is apparent from the above the methods of the present invention are especially suited to be carried out in small scale. With small scale batches of 200 µl to 500 ml per subset, preferably 1 to 10 ml per subset are meant. These can be handled in the above mentioned equipment which itself is commercially available.

The MHC molecules are selected from the group consisting of MHC Class I, MHC Class II, homo-oligomers thereof, hetero oligomers thereof and mixtures of the same. The MHC proteins may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2 -microglobulin. Included in the murine H-2 subunits are the class I H-2K, H-2D, H-2L, and the class II I-Aα, I-Aβ, I-Eα and I-Eβ, and β2-microglobulin. Amino acid sequences of some representative MHC proteins are referenced in EP 812 331. Also included in the scope of this invention are non-classical MHC molecules such as CD1, HLA-E, HLA-F, HLA-G, Qa1 and CD1. The CD1 monomer may instead of the peptide have a lipid such as a glycolipid bound in its groove. The present invention is also applicable to the situation where a lipid instead of a peptide is bound and the skilled worker will be capable of translating the above protocols to this situation.

In a preferred embodiment, the MHC peptide chain proteins correspond to the soluble form of the normally membrane-bound protein. For class I subunits, the soluble form is derived from the native form by deletion of the transmembrane and cytoplasmic domains. For class I proteins, the soluble form will include the α1, α2 and α3 domains of the α chain protein and beta 2 microglobulin, respectively. For class II proteins the soluble form will include the α1 and α2 and β1 and β2 domains of the α chain protein and β chain protein, respectively.

In general, the MHC molecule may in one or more of the proteins chains or peptide comprised therein further comprise one or more additional domains such as one or more linkers, a tagging domain and a purification domain. The additional domain(s) may e.g. be provided on the multivalent entity in case of oligomers or on the peptide, the MHC alpha and/or beta chain proteins, respectively.

The present invention will be further illustrated by way of the following example, which is however not intended to limit the same.

### Example

In this example a set of 24 subsets of MHC I molecules was purified in a method according to the invention.

### Step a):

2 ml small scale refolds of soluble MHC Class I molecules of the allele HLA-A*0201 with 24 different binding peptides are set up according to the protocol as described in Garboci *et al. (supra),* by scaling down the refold method described therein to the volume of interest. Solubilised HLA-A*0201 alpha chain protein and beta 2 microglobulin were obtained as described in the reference with the modification that the alpha chain protein was obtained in a pre-biotinylated form following the protocol described in WO9504069 by fusing a biotinylation peptide to the C-terminal end of the alpha chain protein. Where this protocol uses stirring of the refolding mixture, vortexing is used following preparation of the refolding mixture and after the addition of each of the protein chains and the binding peptide. Refolds are incubated overnight at 4°C.

### Step b):

Following refolding, the above samples are filtered through a 0.22µm syringe filter prior to purification using 24 VivaPure^{®} Q Mini H anion exchange columns. Purification occurred by applying centrifugal force in a microcentrifuge with a 24 place rotor accepting 2ml Eppendorf^{®} microcentrifuge tubes. Specifically, the following steps were applied:
a) Placing of each column onto 2ml Eppendorf^{®} collection tube
b) Equilibration of each column with 400 µl loading buffer (20mM Tris/HCL pH 8.0) and centrifuge for 5 min at 2000 x g in microcentrifuge.
c) Loading of crude refold sample of step a) into the column (up to 400 µl, one batch per tube) and centrifuge for 5 min as above. Loadings were repeated as appropriate until all of the sample from each batch was loaded
d) Resin wash step 1: Addition of 400 µl loading buffer to column and centrifugation for 5 min as above.
e) Resin wash step 2: Addition of 400 µl loading buffer to column and centrifugation for 5 min as above.
f) Sample elution: Addition of 400 µl elution buffer (20mM Tris pH 8.0 + 300mM NaCl) to column and centrifugation for 5 min as above.
g) Placing of each column onto fresh 2ml Eppendorf^{®} collection tube.
h) Recovery pure MHC molecules in the elution buffer at the bottom of the tube.

Following purification, the protein amount and concentration in each sample is determined by the method of Bradford.

Purified biotinylated MHC monomers are now ready for coupling to streptavidin in a variety of applications. In one application they can be coupled to R-PE labelled streptavidin in a 4:1 molar ratio of biotinylated monomer to streptavidin molecule to provide MHC tetramers that can be used to detect antigen specific T cells in flow-cytometry.

## Claims

1. A method of producing a set of MHC molecules, which set includes a plurality of subsets of MHC molecules, wherein the MHC molecules of each subset differ from MHC molecules of at least one other subset in at least one element selected from the group consisting of a peptide bound in the MHC binding groove, an MHC alpha chain protein and an MHC beta chain protein, said method comprising the steps of:
a) providing the MHC molecules for each subset and loading said MHC molecules with said peptide by refolding MHC alpha and beta chain proteins of said MHC molecules in presence of said peptide, and
b) purifying in parallel the subsets of MHC molecules as obtained in step a) by a chromatographic method using a stepped gradient for elution.

2. The method of claim 1, wherein in step a) the alpha and beta chain proteins are expressed in a bacterial host and obtained from inclusion body material.

3. A method of producing a set of MHC molecules, which set includes a plurality of subsets of MHC molecules, wherein the MHC molecules of each subset differ from MHC molecules of at least one other subset in at least one element selected from the group consisting of a peptide bound in the MHC binding groove, an MHC alpha chain protein and an MHC beta chain protein, said method comprising the steps of:
a) providing the MHC molecules for each subset and loading said MHC molecules with said peptide by refolding in presence of said peptide or by peptide exchange,and
b) purifying in parallel the subsets of MHC molecules as obtained in step a) by a chromatographic method using a stepped gradient for elution,
wherein the set comprises at least 4 subsets of MHC molecules.

4. The method of claims 1, 2 or 3, wherein the product of step a) is subjected to step b) without prior concentration.

5. The method of any of claims 1 to 4, wherein the purification step b) comprises the steps of
b1) loading the product of steps a) on an adsorption matrix,
b2) washing the loaded adsorption matrix at least once, and
b3) eluting the adsorption matrix by applying a stepped gradient,
b4) recovering the desired subset of MHC molecules in the eluate of step b3).

6. The method of claim 5, wherein the steps b1) to b4) are repeated, individually or in combination, until the desired purity of the product is obtained.

7. The method of claim 5 or 6, wherein at least one of the steps b1) to b3) is carried out in parallel.

8. The method of any one of claims 1 to 7, wherein the MHC molecules differ with regard to their peptides and are loaded with a peptide specific for the respective subset in step a).

9. The method of claim 8, wherein said peptide is substantially homogeneous for each subset.

10. The method of any of claims 1 or 2, or of claims 4 to 9 in combination with claim 1, wherein the set comprises at least 2 subsets of MHC molecules.

11. The method of claim 3, or claims 4 to 9 in combination with claim 3, wherein the set comprises at least 10 subsets of MHC molecules.

12. The method of any preceding claim, wherein the chromatographic method uses a matrix selected from the group of resins, beads and membranes, in form of a column chromatography.

13. The method of claim 12, wherein the chromatographic method is selected from a group consisting of ion exchange chromatography and ion exchange chromatography using a basic ion exchanger.

14. The method of any preceding claim, wherein the stepped gradient is selected from a salt gradient, a pH gradient or a gradient employing a denaturing agent, and mixtures thereof.

15. The method of claim 14 wherein the stepped gradient is a one-step gradient.

16. The method of any preceding claim, wherein the conditions for carrying out at least one of the steps of purification in b) are identical for at least two subsets.

17. The method of any of claims 3 to 16, wherein the MHC molecules for each subset are provided in step a) by refolding MHC alpha and beta chain proteins of said MHC molecules in the presence of the desired peptide.

18. The method of any one of claims 3 to 17, wherein the MHC molecules for each subset are provided in step a) by exchanging the peptide bound in said MHC molecules under suitable conditions to load said peptide.

19. The method of any preceding claim, wherein the MHC molecules are selected from MHC monomers and oligomers such as dimers to decamers.

20. The method of any preceding claim, wherein the MHC molecules are MHC monomers, which have been biotinylated.

21. The method of claim 20, wherein the MHC monomers have been biotinylated on either their alpha or beta chain protein before loading of the peptide by refolding.

22. The method of claim 20 in combination with claim 18, wherein the MHC monomers have been biotinylated on either their alpha or beta chain protein before loading of the peptide either by refolding or peptide exchange.

23. The method of any of claims 19 to 22, which further includes the step of oligomerizing the MHC monomers to yield the desired oligomer.

24. The method of claim 23, wherein the step of oligomerization occurs before step b) and after step a), or wherein oligomerization occurs in parallel to refolding in step a).

25. The method of any preceding claim, wherein the chromatographic method uses a porous membrane adsorber chromatography matrix.

26. The method of any of the preceding claims, wherein the driving force for at least one of the steps of purification selected from loading, washing and elution is selected from a centrifugal force applied by centrifuging or a pressure drop caused through applying a vacuum.

27. The method of claim 26 wherein the plurality of subsets is purified simultaneously in the same centrifuge or on a vacuum manifold.

28. The method of any one of the preceding claims, wherein a chromatography column is mounted directly on the receptacle receiving the eluate during the elution step

29. The method of claim 28 wherein the receptacle is a microcentrifuge tube of between 0.5 to 2 ml volume.

30. The method of claim 5, wherein the solution used for at least one of the steps of purification selected from loading, washing and elution is an aqueous solution of pH above 7.

31. The method of any of the preceding claims, wherein a salt gradient of at least 100 mM salt is used.

32. The method of any of the preceding claims, wherein the product obtained in step b) is further subjected to one or more steps selected from a labelling reaction, lyophilisation, immobilisation on a solid surface or incorporation into a lipid (bi)layer.

33. The method of one of the preceding claims carried out in small scale of 200 µl to 500 ml per subset.

34. The method of one of the preceding claims, wherein the MHC molecules are selected from the group consisting of MHC Class I, MHC Class II, CD1, HLA-E, HLA-F, HLA-G, homo-oligomers thereof, hetero-oligomers thereof and mixtures of the same.

## Patentansprüche

1. Verfahren zur Herstellung eines Satzes von MHC-Molekülen, welcher Satz eine Mehrzahl an Teilsätzen von MHC-Molekülen beinhaltet,
wobei die MHC-Moleküle jedes Teilsatzes sich von MHC-Molekülen von zumindest einem anderen Teilsatz in zumindest einem Element, ausgewählt aus der Gruppe, bestehend aus einer Peptidbindung in der MHC-Bindungsfurche, einem MHC-α-Ketten-Protein und einem MHC-β-Ketten-Protein, unterscheiden, wobei dieses Verfahren die Schritte umfasst:
a) Bereitstellen der MHC-Moleküle für jeden Teilsatz und Beladung dieser MHC-Moleküle mit diesem Peptid durch Rückfaltung von MHC-α- und -β-Ketten-Proteinen dieser MHC-Moleküle in Anwesenheit von diesem Peptid, und
b) parallele Aufreinigung der Teilsätze von MHC-Molekülen wie in Schritt a) erhalten durch ein chromatographisches Verfahren unter Verwendung eines Stufengradienten zur Elution.

2. Verfahren nach Anspruch 1, wobei in Schritt a) die α- und β-Ketten-Proteine in einem bakteriellen Wirt exprimiert und aus Einschlusskörper-Material gewonnen werden.

3. Verfahren zur Herstellung eines Satzes von MHC-Molekülen, welcher Satz eine Mehrzahl von Teilsätzen von MHC-Molekülen beinhaltet,
wobei die MHC-Moleküle jedes Teilsatzes sich von MHC-Molekülen von zumindest einem anderen Teilsatz in zumindest einem Element, ausgewählt aus der Gruppe, bestehend aus einer Peptidbindung in der MHC-Bindungsfurche, einem MHC-α-Ketten-Protein und einem MHC-β-Ketten-Protein, unterscheiden, wobei dieses Verfahren die Schritte umfasst:
a) Bereitstellen der MHC-Moleküle für jeden Teilsatz und Beladung dieser MHC Moleküle mit diesem Peptid durch Rückfaltung in Anwesenheit von diesem Peptid oder durch Peptidaustausch, und
b) parallele Aufreinigung der Teilsätze von MHC-Molekülen wie in Schritt a) erhalten durch ein chromatographisches Verfahren unter Verwendung eines Stufengradienten zur Elution,
wobei der Satz zumindest 4 Teilsätze von MHC-Molekülen umfasst.

4. Verfahren nach Anspruch 1, 2 oder 3, wobei das Produkt von Schritt a) ohne vorherige Konzentration dem Schritt b) unterzogen wird.

5. Verfahren nach einem der Ansprüche 1 bis 4, wobei der Aufreinigungsschritt b) die folgenden Schritte umfasst:
b1) Laden des Produktes aus den Schritten a) auf eine Adsorptionsmatrix,
b2) zumindest einmaliges Waschen der beladenen Adsorptionsmatrix, und
b3) Eluieren der Adsorptionsmatrix durch Anlegen eines Stufengradienten,
b4) Wiedergewinnung des gewünschten Teilsatzes von MHC-Molekülen in dem Eluat von Schritt b3).

6. Verfahren nach Anspruch 5, wobei die Schritte b1) bis b4) einzeln oder in Kombination wiederholt werden bis die gewünschte Reinheit des Produktes erzielt ist.

7. Verfahren nach Anspruch 5 oder 6, wobei zumindest einer der Schritte b1) bis b3) parallel durchgeführt wird.

8. Verfahren nach einem der Ansprüche 1 bis 7, wobei sich die MHC-Moleküle hinsichtlich ihrer Peptide unterscheiden und mit einem für den zugehörigen Teilsatz in Schritt a) spezifischen Peptid beladen sind.

9. Verfahren nach Anspruch 8, wobei dieses Peptid für jeden Teilsatz im Wesentlichen homogen ist.

10. Verfahren nach einem der Ansprüche 1 oder 2, oder nach einem der Ansprüche 4 bis 9 in Kombination mit Anspruch 1, wobei der Satz zumindest 2 Teilsätze von MHC-Molekülen umfasst.

11. Verfahren nach Anspruch 3, oder nach den Ansprüchen 4 bis 9 in Kombination mit Anspruch 3, wobei der Satz zumindest 10 Teilsätze von MHC-Molekülen umfasst.

12. Verfahren nach einem der vorhergehenden Ansprüche, wobei das chromatographische Verfahren eine Matrix, ausgewählt aus der Gruppe von Harzen, Kügelchen und Membranen, in Form einer Säulenchromatographie nutzt.

13. Verfahren nach Anspruch 12, wobei das chromatographische Verfahren aus einer Gruppe, bestehend aus Ionenaustauschchromatographie und Ionenaustauschchromatographie mittels eines basischen Ionenaustauschers, ausgewählt ist.

14. Verfahren nach einem der vorhergehenden Ansprüche, wobei der Stufengradient aus einem Salzgradienten, einem pH-Gradienten oder einem Gradienten unter Einsatz eines denaturierenden Agens und Mischungen hieraus ausgewählt ist.

15. Verfahren nach Anspruch 14, wobei der Stufengradient ein Ein-Stufen-Gradient ist.

16. Verfahren nach einem der vorhergehenden Ansprüche, wobei die Bedingungen für die Durchführung zumindest eines der Aufreinigungsschritte in b) für zumindest zwei Teilsätze identisch sind.

17. Verfahren nach einem der Ansprüche 3 bis 16, wobei die MHC-Moleküle für jeden Teilsatz in Schritt a) durch Rückfaltung von MHC-α- und -β-Ketten-Proteinen dieser MHC-Moleküle in Gegenwart des gewünschten Peptids bereitgestellt werden.

18. Verfahren nach einem der Ansprüche 3 bis 17, wobei die MHC-Moleküle für jeden Teilsatz in Schritt a) durch Austausch der Peptidbindung in diesen MHC-Molekülen unter zur Ladung dieses Peptids geeigneten Bedingungen bereitgestellt werden.

19. Verfahren nach einem der vorhergehenden Ansprüche, wobei die MHC-Moleküle aus MHC-Monomeren und -Oligomeren wie Dimeren bis Decameren ausgewählt werden.

20. Verfahren nach einem der vorhergehenden Ansprüche, wobei die MHC-Moleküle MHC-Monomere sind, welche biotinyliert worden sind.

21. Verfahren nach Anspruch 20, wobei die MHC-Monomere vor dem Laden des Peptids durch Rückfaltung entweder an ihrem α- oder β-Ketten-Protein biotinyliert worden sind.

22. Verfahren nach Anspruch 20 in Kombination mit Anspruch 18, wobei die MHC-Monomere vor dem Laden des Peptids entweder durch Rückfaltung oder durch Peptidaustausch entweder an ihrem α- oder β-Ketten-Protein biotinyliert worden sind.

23. verfahren nach einem der Ansprüche 19 bis 22, welches zusätzlich den Schritt der Oligomerisierung der MHC-Monomere beinhaltet, um das gewünschte Oligomer zu ergeben.

24. Verfahren nach Anspruch 23, wobei der Schritt der Oligomerisierung vor Schritt b) und nach Schritt a) erfolgt oder
wobei die Oligomerisierung parallel mit der Rückfaltung in Schritt a) erfolgt.

25. Verfahren nach einem der vorhergehenden Ansprüche, wobei das chromatographische Verfahren eine poröse Membran-Adsorptionschromatographie-Matrix nutzt.

26. Verfahren nach einem der vorhergehenden Ansprüche, wobei die treibende Kraft für zumindest einen der Aufreinigungsschritte, ausgewählt aus Beladung, Waschen und Elution, aus einer mittels Zentrifugation erzeugten Zentrifugalkraft oder mittels eines durch Anlegen eines Vakuums verursachten Druckabfalls ausgewählt ist.

27. Verfahren nach Anspruch 26, wobei die Mehrzahl der Teilsätze gleichzeitig in derselben Zentrifuge oder an einem Vakuum-Anschluss aufgereinigt wird.

28. Verfahren nach einem der vorhergehenden Ansprüche, wobei eine Chromatographiesäule direkt an dem das Eluat während des Elutionsschrittes empfangenden Behälter befestigt ist.

29. Verfahren nach Anspruch 28, wobei der Behälter ein Mikrozentrifugationsröhrchen mit einem Volumen zwischen 0,5 und 2 ml ist.

30. Verfahren nach Anspruch 5, wobei die für zumindest einen der Aufreinigungsschritte, ausgewählt aus Beladung, Waschen und Elution, verwendete Lösung eine wässrige Lösung mit einem pH-Wert von über 7 ist.

31. Verfahren nach einem der vorhergehenden Ansprüche, wobei ein Salzgradient mit zumindest 100 mM Salz eingesetzt wird.

32. Verfahren nach einem der vorhergehenden Ansprüche, wobei das in Schritt b) erhaltene Produkt einem oder mehreren weiteren Schritten, ausgewählt aus einer Markierungsreaktion, Lyophilisierung, Immobilisierung auf einer festen Oberfläche oder Einschluss in eine Lipid-(Doppel)schicht, unterzogen wird.

33. Verfahren nach einem der vorhergehenden Ansprüche, durchgeführt in kleinem Maßstab von 200 µl bis 500 µl pro Teilsatz.

34. Verfahren nach einem der vorhergehenden Ansprüche, wobei die MHC-Moleküle aus der Gruppe, bestehend aus MHC Klasse I, MHC Klasse II, CD1, HLA-E, HLA-F, HLA-G, Homo-Oligomeren hieraus, Hetero-Oligomeren hieraus und Mischungen derselben, ausgewählt sind.

## Revendications

1. Procédé de production d'un ensemble de molécules du CMH, lequel ensemble comprend une pluralité de sous-ensembles de molécules du CMH dans lequel les molécules du CMH de chaque sous-ensemble diffèrent des molécules du CMH d'au moins un autre sous-ensemble en au moins un élément sélectionné dans le groupe consistant en un peptide lié dans le sillon de liaison au CMH, une protéine du CMH de chaîne alpha et une protéine du CMH de chaîne bêta, ledit procédé comprenant les étapes de :
a) préparer les molécules du CMH pour chaque sous-ensemble et charger lesdites molécules du CMH avec ledit peptide en repliant les protéines du CMH de chaîne alpha et de chaîne bêta desdites molécules du CMH en présence dudit peptide, et
b) purifier parallèlement les sous-ensembles des molécules du CMH telles qu'elles ont été obtenues à l'étape a) par un procédé chromatographique utilisant un gradient étagé pour élution.

2. Procédé selon la revendication 1 dans lequel dans l'étape a) les protéines de chaîne alpha et de chaîne bêta sont exprimées dans un hôte bactérien et sont obtenues de matériau de corps d'inclusion.

3. Procédé de production d'un ensemble de molécules du CMH, lequel ensemble comprend une pluralité de sous-ensembles de molécules du CMH dans lequel les molécules du CMH de chaque sous-ensemble diffèrent des molécules du CMH d'au moins un autre sous-ensemble en au moins un élément sélectionné dans le groupe consistant en un peptide lié dans le sillon de liaison au CMH, une protéine du CMH de chaîne alpha et une protéine du CMH de chaîne bêta, ledit procédé comprenant les étapes de :
a) préparer les molécules du CM pour chaque sous-ensemble et charger lesdites molécules du CMH avec ledit peptide par repliage en présence dudit peptide ou par échange de peptide, et
b) purifier parallèlement les sous-ensembles des molécules du CMH telles qu'elles ont été obtenues à l'étape a) par un procédé chromatographique en utilisant un gradient étagé pour élution,
dans lequel l'ensemble comprend au moins 4 sous-ensembles de molécules du CMH.

4. Procédé selon les revendications 1, 2 ou 3 dans lequel le produit de l'étape a) est soumis à l'étape b) sans concentration préalable.

5. Procédé selon l'une quelconque des revendications 1 à 4 dans lequel l'étape de purification b) comprend les étapes de
b1) charger le produit des étapes a) sur une matrice d'adsorption,
b2) laver la matrice d'adsorption chargée au moins une fois, et
b3) éluer la matrice d'adsorption en appliquant un gradient étagé,
b4) récupérer le sous-ensemble désiré de molécules du CMH dans l'éluat de l'étape b3).

6. Procédé selon la revendication 5 dans lequel les étapes b1) à b4) sont répétées, individuellement ou en combinaison, jusqu'à ce que la pureté désirée du produit soit obtenue.

7. Procédé selon la revendication 5 ou 6 dans lequel au moins l'une des étapes b1) à b3) est exécutée parallèlement.

8. Procédé selon l'une quelconque des revendications 1 à 7 dans lequel les molécules du CMH diffèrent en ce qui concerne leurs peptides et sont chargées avec un peptide spécifique pour le sous-ensemble respectif dans l'étape a).

9. Procédé selon la revendication 8 dans lequel ledit peptide est substantiellement homogène pour chaque sous-ensemble.

10. Procédé selon l'une quelconque des revendications 1 ou 2 ou des revendications 4 à 9 en combinaison avec la revendication 1 dans lequel l'ensemble comprend au moins 2 sous-ensembles de molécules du CMH.

11. Procédé selon la revendication 3 ou les revendications 4 à 9 en combinaison avec la revendication 3 dans lequel l'ensemble comprend au moins 10 sous-ensembles de molécules du CMH.

12. Procédé selon l'une quelconque des revendications précédentes dans lequel le procédé chromatographique utilise une matrice sélectionnée dans le groupe des résines, des billes et membranes, sous forme de chromatographie sur colonne.

13. Procédé selon la revendication 12 dans lequel le procédé chromatographique est sélectionné dans un groupe comprenant la chromatographie échangeuse d'ions et la chromatographie échangeuse d'ions qui utilise un échangeur d'ions basique.

14. Procédé selon l'une quelconque des revendications précédentes dans lequel le gradient étagé est sélectionné parmi un gradient de sel, un gradient de pH ou un gradient qui emploie un agent dénaturant et des mélanges de ceux-ci.

15. Procédé selon la revendication 14 dans lequel le gradient étagé est un gradient à un étage.

16. Procédé selon l'une quelconque des revendications précédentes dans lequel les conditions pour exécuter au moins l'un des étages de purification dans b) sont identiques pour au moins deux sous-ensembles.

17. Procédé selon l'une quelconque des revendications 3 à 16 dans lequel les molécules du CMH pour chaque sous-ensemble sont préparées dans l'étape a) en repliant les protéines du CMH de chaîne alpha et de chaîne bêta desdites molécules du CMH en présence du peptide désiré.

18. Procédé selon l'une quelconque des revendications 3 à 17 dans lequel les molécules du CMH pour chaque sous-ensemble sont préparées dans l'étape a) en échangeant le peptide lié dans lesdites molécules du CMH dans des conditions appropriées pour charger ledit peptide.

19. Procédé selon l'une quelconque des revendications précédentes dans lequel les molécules du CMH sont sélectionnées parmi les monomères et les oligomères du CMH tels que les dimères à décamères.

20. Procédé selon l'une quelconque des revendications précédentes dans lequel les molécules du CMH sont des monomères du CMH qui ont été biotinylés.

21. Procédé selon la revendication 20 dans lequel les monomères du CMH ont été biotinylés sur soit leur protéine de chaîne alpha ou bêta avant chargement du peptide par repliage.

22. Procédé selon la revendication 20 en combinaison avec la revendication 18 dans lequel les monomères de CMH ont été biotinylés sur soit leur protéine de chaîne alpha ou chaîne bêta avant chargement du peptide par repliage ou par échange de peptide.

23. Procédé selon l'une quelconque des revendications 19 à 22 qui comprend de plus l'étape d'oligomérisation des monomères du CMH pour produire l'oligomère désiré.

24. Procédé selon la revendication 23 dans lequel l'étape d'oligomérisation se fait avant l'étape b) et après l'étape a) ou dans lequel l'oligomérisation se fait en parallèle avec le repliage dans l'étape a).

25. Procédé selon l'une quelconque des revendications précédentes dans lequel le procédé chromatographique utilise une matrice de chromatographie à adsorbeur à membrane poreuse.

26. Procédé selon l'une quelconque des revendications précédentes dans lequel la force motrice pour au moins l'une des étapes de purification sélectionnées dans le chargement, le lavage et l'élution est sélectionnée dans une force centrifuge appliquée par centrifugation ou par une chute de pression causée en appliquant un vide.

27. Procédé selon la revendication 26 dans lequel la pluralité de sous-ensembles est purifiée simultanément dans la même centrifugeuse ou sur un distributeur à vide.

28. Procédé selon l'une quelconque des revendications précédentes dans lequel une colonne de chromatographie est montée directement sur le réceptacle qui reçoit l'éluat pendant l'étape d'élution.

29. Procédé selon la revendication 28 dans lequel le réceptacle est un tube de microcentrifugation d'un volume entre 0,5 à 2 ml.

30. Procédé selon la revendication 5 dans lequel la solution utilisée pour au moins l'une des étapes de purification sélectionnées dans le chargement, le lavage et l'élution est une solution aqueuse de pH supérieur à 7.

31. Procédé selon l'une quelconque des revendications précédentes dans lequel un gradient de sel d'au moins 100 mM de sel est utilisé.

32. Procédé selon l'une quelconque des revendications précédentes dans lequel le produit obtenu à l'étape b) est de plus soumis à une ou plusieurs étapes sélectionnées parmi une réaction de marquage, la lyophilisation, l'immobilisation sur une surface solide ou l'incorporation dans une (bi)couche de lipides.

33. Procédé selon l'une des revendications précédentes exécuté à petite échelle de 200 µl à 500 µl par sous-ensemble.

34. Procédé selon l'une des revendications précédentes dans lequel les molécules du CMH sont sélectionnées dans le groupe consistant en des molécules du CMH de classe I, du CMH de classe II, CD1, HLA-E, HLA-F, HLA-G, des homo-oligomères de celles-ci, des hétéro-oligomères de celles-ci et des mélanges de celles-ci.
